# EUROPÄISCHE PATENTSCHRIFT

(11) **EP 4 737 884 B1**
(45) Veröffentlichungstag und Bekanntmachung des Hinweises auf die Patenterteilung: **30.09.2026**
(21) Anmeldenummer: 24209437.3
(22) Anmeldetag: 29.10.2024
(51) Int. Cl.: G01N 21/3554, G01N 21/359, G01N 21/3563, G01N 33/46

(54) **VERFAHREN ZUR VORHERSAGE DER BENETZBARKEIT DER OBERFLÄCHE EINER HOLZWERKSTOFFPLATTE**
METHOD FOR PREDICTING WETTABILITY OF THE SURFACE OF A WOOD-BASED MATERIAL BOARD
PROCÉDÉ DE PRÉDICTION DE LA CAPACITÉ DE MOUILLABILITÉ DE SURFACE DE PLAQUE EN BOIS

(43) Veröffentlichungstag der Anmeldung: 06.05.2026
(73) Patentinhaber: Flooring Technologies Ltd., Kalkara SCM1001 (MT)
(72) Erfinder: KALWA, Norbert, 32805 Horn-Bad Meinberg (DE); ZHANG, Jinming, 10247 Berlin (DE); SEIDACK, Georg, 16909 Wistock/ Dosse (DE)
(74) Vertreter: Maikowski & Ninnemann Patentanwälte Partnerschaft mbB

(56) Entgegenhaltungen:
- EP-B1- 2 915 658
- EP-B1- 4 191 230
- US-A- 5 680 321
- US-A1- 2004 094 851
- US-A1- 2016 123 871
- POVER R ET AL: "NIR-FEUCHTEANALYSATOREN FUER BERUEHRUNGSLOSE ON-LINE-MESSUNGEN UNTER PRODUKTIONSBEDINGUNGEN", TM - TECHNISCHES MESSEN/PLATTFORM FÜR METHODEN, SYSTEME UND ANWENDUNGEN DER MESSTECHNIK, R.OLDENBOURG VERLAG. MUNCHEN, DE, vol. 59, no. 3, March 1992 (1992-03-01), pages 116 - 120, XP000297912, ISSN: 0171-8096

## Beschreibung

Die vorliegende Erfindung betrifft ein Verfahren zur Vorhersage der Beschichtbarkeit bzw. Benetzbarkeit der Oberfläche einer Holzwerkstoffplatte und die Verwendung der mittels dieses Verfahrens gewonnenen Parameter.

Bei der Herstellung von dekorativen Oberflächen auf Holzwerkstoffen kommen seit Jahrzehnten unterschiedliche Technologien zum Einsatz. Während man zunächst einfache unifarbene Dekore oder Zweifarbdekore direkt auf die Platte aufbrachte, kamen mit der Kurztaktpressen-Technologie auch hochwertige Drucke auf Papier zum Einsatz.

Dabei hat sich in den letzten Jahren wieder eine Tendenz zu einer Direktbedruckung dieser Oberflächen entwickelt, auch getrieben durch die Fortschritte, die bei der Digitaldruck-Technologie zu beobachten sind. Wobei diese Technologie anders als in der Vergangenheit mehr und mehr lösemittelfrei ist.

Diese Technologien kommen auf Anlagen zum Einsatz, die mit hohen Geschwindigkeiten mehrere hunderttausend Quadratmeter pro Tag produzieren können. Bei derartig hohen Produktionsgeschwindigkeiten muss natürlich gewährleistet sein, dass der Prozess nicht durch Probleme der Vorprodukte gestört wird.

Ein wesentlicher Punkt ist dabei, dass die Grundierung der Holzwerkstoff-Platte (HWS-Platte) qualitativ einwandfrei ist. Qualitativ einwandfrei bedeutet dabei, dass die Grundierung sowohl von der Menge als auch von der Verteilung über die gesamte Platte im Rahmen der Vorgaben liegt. Dies gilt, wie sich gezeigt hat, insbesondere bei digital aufgedruckten Dekoren. Hier können bereits geringe Variationen in der (Farb)grundierung zu deutlich sichtbaren Farbunterschieden führen. Wegen der hohen Produktionsgeschwindigkeiten werden die Farbunterschiede allerdings bestenfalls frühstens nach dem Druck bemerkt. Dann sind aber schon eine größere Menge an Platten in der Produktionslinie. Teilweise wurde der Farbunterschied auch erst nach der Produktion oder einem weiteren Produktionsschritt festgestellt. Dann waren unter Umständen aber schon mehrere hundert Platten bedruckt.

Eine Maßnahme zur Verarbeitung von kritischen Plattenchargen war, dass die Produktionsgeschwindigkeit signifikant reduziert wurde (bis zu 20%). Jedoch waren bis zu dem Zeitpunkt an dem die Probleme bemerkt wurden, schon größere Mengen an Platten durch die Produktionslinie hindurch gelaufen.

Die Ursachen für die teilweise qualitativ nicht einwandfreien Drucke blieben dabei lange unklar. Dies hatte auch damit zu tun, dass sich dieses Phänomen messtechnisch nicht analysieren ließ. Bekannte Analysemethoden, die für derartige Produkte/Probleme existieren, wurden angewendet. Eine Bestimmung der Oberflächenspannung lieferte bei Platten, die Druckprobleme zeugten, keine Unterschiede zu unauffälligen Platten. Auch Prüfungen bei denen über Lauftests mit verschiedenen Flüssigkeiten (Toluol, Wasser) das Eindringverhalten in den Träger geprüft wird, lieferten keine eindeutigen Hinweise auf Unterschiede in den Plattenoberflächen.

Auch Densogramme von Platten aus den kritischen Chargen zeigten keine Unterschiede zu Platten aus unkritischen Chargen. Auch die Bestimmung der Plattenfeuchte über die Darrprobe (Trocknung bei 105°C, 24 h) lieferte keine gesicherten Zusammenhänge. Der Grund hierfür ist, dass ein Feuchtewert einer Platte keine Aussage über die Verteilung der Feuchte über den Querschnitt liefert. Es wurde vermutet, dass die Feuchte der Deckschicht der Platte der endscheidende Einflussparameter für die Grundierbarkeit war. Allerdings war bisher kein Verfahren zur Bestimmung dieser Feuchte bekannt. Ein mögliches Verfahren zur Bestimmung der Feuchte in der Deckschicht, wäre ein Abtrennen der Deckschichten mit Hilfe einer Säge. Allerdings führt das Abtrennen der Deckschichten z. B. mit Hilfe einer Säge zu einem Wärmeeintrag, der den Messwert mehr oder weniger deutlich verändern kann. Zudem müsste eine relativ dünne Schicht abgetrennt werden, was technisch sehr aufwändig wäre. Auch würde eine derartige Vorgehensweise viel Zeit benötigen ( Probenahme, Zuschnitt, Abtrennen der Deckschicht und Analyse ). Dies wäre in einem laufenden Produktionsbetrieb also nur schwer realisierbar. Dokumente EP4191230B1, EP2915658B1, US2016/123871A1 und Pover R. et Al.: "NIR-Feuchteanalysatoren für Berührungslose Online-Messungen unter Produktionsbedingungen", TM-Technisches Messen/Plattform für Methoden, Systeme und Anwendungen der Messtechnik, R.Oldenbourg Verlag, München, DE, Bd. 59, Nr. 3, März 1992, Seiten 116-120, ISSN: 0171-8096 offenbaren ein Online-Verfahren zur Vorhersage der Feuchte der Oberfläche einer Holzwerksstoffplatte mittels NIR-Spektroskopie. US5680321A offenbart ein Online-Verfahren zur Vorhersage der Benetzbarkeit eines Blattes Papier mittels NIR-Spektroskopie.

Grundsätzlich wurde bei zu "frischen" Platten (d.h. Platten die unmittelbar aus der Presse kommen) ein Problem häufiger beobachtet als bei abgelagerten Platten. Allerdings war eine genaue Definition von zu "frischen" Platten nicht möglich, da dies auch jahreszeitlich variierte (d.h. in Abhängigkeit von dem zur Herstellung der Holzwerkstoffplatten verwendeten Holzmaterialien, den Produktions- und Lagerbedingungen). Zudem musste bei hohen Abnahmemengen auch von Fall zu Fall auf frische Platten zurückgegriffen werden.

Die sich daraus ergebenen Nachteile sind hohe Ausschussmengen, Produktionsungewissheit und eine mögliche Störung des Produktionsablaufs.

Der Erfindung liegt daher die technische Aufgabe zu Grunde ein Messverfahren zu entwickeln mit dem man sicher vorhersagen kann, ob eine Platte qualitativ einwandfrei grundiert bzw. bedruckt, d.h. beschichtet oder benetzt, werden kann. Dies sollte eine Voraussage erlauben bevor die Platten in die Produktionslinie einlaufen. Auch sollte das Ergebnis der Voraussage eine Steuerung der Anlage bezüglich der Produktionsgeschwindigkeit bzw. bei qualitativ nicht einwandfreien Platten eine Ausschleusung von Platten an der Anlage ermöglichen. Im Extremfall sollte auch die Weiterverarbeitung von Platten gestoppt werden.

Es sollte eine zerstörungsfreie Prüfung sein, die kontinuierliche Messwerte liefert und den Produktionsprozess nicht stört. Die Messungen sollten mit hoher Frequenz erfolgen, um der hohen Anlagengeschwindigkeit Rechnung zu tragen. Auch sollte durch die Verwendung eines über die Breite der Produktionslinie verfahrbaren Messkopf, die gezielte Prüfung von problematischen Bereichen (z. B. Plattenränder ) möglich sein.

Diese Aufgabe wird erfindungsgemäß durch ein Verfahren mit den Merkmalen des Anspruchs 1 gelöst.

Entsprechend wird ein Online-Verfahren zur Vorhersage der Beschichtbarkeit bzw.

Benetzbarkeit der Oberfläche mit einem Benetzungsmittel einer Holzwerkstoffplatte bereitgestellt, wobei als Parameter für die Beschichtbarkeit / Benetzbarkeit der Oberfläche mit dem Benetzungsmittel der Holzwerkstoffplatte die Feuchte der Oberfläche der Holzwerkstoffplatte mittels NIR-Spektroskopie (Nahinfrarot-Spektroskopie) bestimmt wird und durch Vergleich mit korrespondierenden Benetzungsgraden aus einem mit Referenzproben erstellten Referenzmodell eine Benetzungsbarkeit der Holzwerkstoffplatte mit dem Benetzungsmittel zugeordnet wird.

Unter der Oberflächenfeuchte ist vorliegend dabei die Feuchte in der obersten Schicht (Deckschicht) der Holzwerkstoffplatte bis zu einer Eindringtiefe von max. 0,2 mm zu verstehen. Vorteilhafterweise wird bei der Messung mit Hilfe von NIR fast genau dieser Tiefenbereich abgedeckt.

Überraschenderweise hat sich gezeigt, dass es zwischen der Oberflächenfeuchte (bzw. Feuchte in der Deckschicht) einer Holzwerksstoffplatte und dem Benetzungsgrad der Oberfläche mit einem Benetzungsmittel (z.B. einem Formaldehyd-Harz) eine Korrelation besteht: je höher der Feuchtegrad desto höher die Benetzbarkeit und desto besser die Beschichtbarkeit der Oberfläche der Holzwerkstoffplatte z.B. mit einer Grundierungsschicht. Die Messung und Bestimmung der Oberflächenfeuchte mittels NIR-Spektroskopie erlaubt zudem ein schnelles und kontinuierliches Verfahren zur Vorhersage, ob sich eine Holzwerkstoffplatte für eine weitergehende Beschichtung eignet oder nicht.

In einer Ausführungsform umfasst das vorliegende Verfahren die folgenden Schritte:
- Bereitstellen von Holzwerkstoffplatten mit jeweils unterschiedlich definierter Oberflächenfeuchte als Referenzproben,
- Aufnahme von mindestens einem NIR-Spektrum von jeder dieser Referenzproben unter Verwendung von mindestens einem NIR-Messkopf in einem Wellenlängenbereich zwischen 1000 nm und 2000 nm, bevorzugt zwischen 1400 nm und 1600, insbesondere bevorzugt zwischen 1400 nm und 1550 nm,
- Zuordnen der unterschiedlichen definierten Oberflächenfeuchten der Referenzproben zu den aufgenommenen NIR-Spektren der jeweiligen Referenzproben,
- Auftragen eines Benetzungsmittels auf die Oberfläche jeder dieser vermessenen Referenzproben mit jeweils unterschiedlich definierter Oberflächenfeuchte und Bestimmen des jeweiligen Benetzungsgrades; d.h. Zuordnen des jeweiligen Benetzungsgrades zu der jeweiligen Oberflächenfeuchte;
- Erstellen eines Kalibriermodells für den Zusammenhang zwischen den Daten der aufgenommenen NIR-Spektren und den dazugehörigen Oberflächenfeuchten als Maß für die dazu korrespondierenden Benetzungsgrade mittels einer multivariaten Datenanalyse;
- Bereitstellen von mindestens einer Holzwerkstoffplatte mit unbekannter Oberflächenfeuchte;
- Aufnehmen von mindestens einem NIR-Spektrum der mindestens einen Holzwerkstoffplatte mit unbekannter Oberflächenfeuchte unter Verwendung von mindestens einem NIR-Messkopf in einem Wellenlängenbereich zwischen 1000 nm und 2000 nm, bevorzugt zwischen 1400 nm und 1600, insbesondere bevorzugt zwischen 1400 nm und 1550 nm, und
- Bestimmen der Oberflächenfeuchte als Maß für die dazu korrespondierenden Benetzungsgrade durch Vergleich des für die Holzwerkstoffplatte aufgenommenen NIR-Spektrums mit dem erstellten Kalibriermodell.

Das vorliegende Verfahren erlaubt eine kontinuierliche Messung der Feuchte der Plattenoberfläche, bevorzugt beim bzw. nach dem Schritt der Plattenvereinzelung. Die Messung kann falls nötig auf traversierend über Plattenbreite und -länge erfolgen. Für die Erstellung des Kalibriermodells werden zunächst Holzwerkstoffplatten-Platten mit definierten Feuchten als Referenzproben mit Hilfe von NIR-Spektroskopie vermessen.

Dann wird mit Hilfe eines wässrigen Harzes - bei Bedarf eingefärbt - die Benetzung der Oberfläche der mehr oder weniger feuchten Oberfläche bestimmt. Dies erfolgt über den Auftrag einer definierten Menge an Harz auf die feuchte Oberfläche des Holzwerkstoffplatte mit einem Rakel. Die Bestimmung der Benetzung erfolgt über eine visuelle Abschätzung. Dieser Prozess kann mehrmals wiederholt werden, um einen möglichen Fehler zu reduzieren. Die Grenze der Benetzbarkeit (bzw. Grundierbarkeit) wird dabei zunächst mit einem Wert von 80% festgelegt, d. h. bei einem Oberflächenfeuchte-Wert bei dem beim Rakelauftrag noch 80% der Oberfläche mit Harz bedeckt sind, wird davon ausgegangen, dass diese Platten an der Produktionslinie keine Fehlgrundierungen aufweisen. Dieser Wert wurde empirisch an der Produktionslinie ermittelt.

Anschließend wird aus den Daten ein Kalibrationsmodell entwickelt mit dessen Hilfe die Oberflächenfeuchte und die damit korrelierende Benetzbarkeit von unbekannten Proben von Holzwerkstoffplatten analysiert werden können.

Je nach Art des verwendeten Benetzungsmittels (Harz, Leim, Lack, die als Lösemittel Wasser enthalten) , kann das Kalibrationsmodell beliebig erweitert werden.. Auch Gemische von Harzen/Leimen als Benetzungsmittel können kalibriert werden. Dabei müssen natürlich den unterschiedliche Feststoffgehalten, Viskositäten, Benetzungen usw. bei der Erarbeitung der Kalibrationsmodelle Rechnung getragen werden.

Mit dem vorliegenden Verfahren ist es nunmehr möglich, die Beschichtbarkeit bzw. Benetzbarkeit einer Holzwerkstoffplatte sicher vorherzusagen, wodurch der Ausschuss und damit die Kosten reduziert werden. Zudem ergibt sich die Möglichkeit zur on-line Anlagensteuerung, wie noch weiter unten erläutert wird.

Die mit dem vorliegenden Verfahren mögliche Bestimmung der Oberflächenfeuchte von Holzwerkstoffplatten erfolgt bevorzugt ausschließlich mittels NIR-Messung. Eine Kombination mit anderen spektroskopischen Methoden, insbesondere unter Verwendung von anderen Wellenlängen außerhalb des NIR-Bereiches ist nicht vorgesehen.

Erfindungsgemäß erfolgt somit der Einsatz eines NIR-Messkopfes, bevorzugt eines NIR-Multimesskopfes, der über die Aufnahme von spektralen Daten (Spektren) im nahinfraroten Bereich (bevorzugt 1400 - 1600 nm) eine Bestimmung der Oberflächenfeuchte zulässt.

Während der Wechselwirkung der NIR-Strahlung mit der Oberfläche der Holzwerkstoffplatte wird die NIR-Strahlung durch die gemessene Probe gestreut und reflektiert. Durch den Empfang der reflektierten NIR-Strahlung per NIR-Detektor wird ein NIR-Spektrum erzeugt. Bei dieser Messung werden in einer Sekunde eine Vielzahl von einzelnen NIR-Messungen durchgeführt, sodass auch eine statistische Absicherung der Werte gewährleistet wird. Die NIR-Spektroskopie zusammen mit der (unten angeführten) Multivariaten Datenanalyse bietet eine Möglichkeit an, einen direkten Bezug zwischen den spektralen Informationen (NIR-Spektren) und den zu bestimmenden Parametern - wie vorliegend der Oberflächenfeuchte der Holzwerkstoffplatte - herzustellen.

Zu beachten ist auch, dass die Referenzprobe gleichartig zu der zu vermessenden Probe ist, d.h. dass die als Referenzproben verwendeten Holzwerkstoffplatten und die zu vermessenden Holzwerkstoffplatten aus Prozessen mit ähnlichen Randbedingungen (insbesondere Pressdruck, Temperatur, Presshaut) stammen.

Von diesen Referenzproben werden zumindest ein NIR-Spektrum in einem Wellenlängenbereich zwischen 1000 nm und 2000 nm, bevorzugt zwischen 1400 nm und 1600, insbesondere bevorzugt zwischen 1400 nm und 1550 nm aufgenommen.

Die unterschiedlichen Oberflächenfeuchten der Referenzproben werden dann den jeweils aufgenommen NIR-Spektren dieser Referenzproben zugeordnet. Des Weiteren wird den vermessenen Referenzproben mit jeweils unterschiedlich definierter Oberflächenfeuchte ein Benetzungsgrad zugeordnet, der (manuell) durch Auftrag eines Benetzungsmittels auf die Oberfläche der jeweiligen Holzwerkstoffplatte bestimmt wird. Es wird ein Kalibriermodell für den Zusammenhang zwischen den spektralen Daten der NIR-Spektren der Referenzproben und den dazugehörigen Oberflächenfeuchte als Parameterwert für den Benetzungsgrad mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens eine Messprobe aus einer Holzwerkstoffplatte mit unbekannter Oberflächenfeuchte bereitgestellt, und mindestens ein NIR-Spektrum dieser Messprobe aufgenommen. Die Oberflächenfeuchte und die damit korrespondierende Benetzbarkeit kann durch Vergleich des für die Messprobe aufgenommenen NIR-Spektrums mit dem erstellten Kalibriermodell ermittelt werden.

Ein Vergleich und die Interpretation der NIR-Spektren erfolgt sinnvollerweise über den gesamten aufgenommenen Spektralbereich, bevorzugt in einem Bereich zwischen 1400 nm und 1600, insbesondere bevorzugt zwischen 1400 nm und 1550 nm. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

Die Bedeutung einer Wellenlänge für die Vorhersage von Parametern, wie der Oberflächenfeuchte, aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell für die Bestimmung der Oberflächenfeuchte, dass der Wellenlängenbereich zwischen 1400 nm und 1600 nm, besonders bevorzugt zwischen 1400 und 1550 nm, für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen (wie Transparenz des Bindemittels, Oberflächenbeschaffenheit der Holzpartikel usw.) zu berücksichtigen bzw. zu minimieren.

Zur Eliminierung von störenden Einflüssen (wie z.B.: Beschaffenheit der Oberfläche der Holzwerkstoffplatte, Farbe) ist es notwendig, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten.

In einer Variante des vorliegenden Verfahrens kann die zu vermessende Oberfläche der Holzwerkstoffplatte mit einer Presshaut oder Verrottungsschicht versehen sein. Diese Presshaut entsteht bei der Herstellung der Holzwerkstoffplatten in den Heißpressen durch den direkten Kontakt der beleimten Partikel und Fasern mit den heißen Pressbändern bei kontinuierlichen Pressen. Bei diesem Kontakt vercracken die Holzfasern und der Leim. Die Presshaut oder verrottete Zone hat eine Dicke von etwa 0,1 - 0,2 mm. Diese Presshaut führt zu einer Schwachzone in der Deckschicht. Diese Schwachzone ist in einem Densogramm der Platte als Rohdichteabfall erkennbar. Entsprechend kann die Presshaut einen Einfluss auf die Oberflächenfeuchte der Holzwerkstoffplatte haben.

Es ist aber auch möglich, dass die Presshaut oder Verrottungsschicht vor der weiteren Verarbeitung der Holzwerkstoffplatte mittels Schleifen entfernt wird. Der Schleifprozess wird üblicherweise nach einer kurzen Abkühlphase der Trägerplatten nach Verlassen der Presseinheit direkt nach der Herstellung der Holzwerkstoffplatte durchgeführt. Der Schleifprozess bewirkt ein Abtragen der auf der Ober- und Unterseite der Holzwerkstoffplatten nach dem Verpressen ausgebildeten Presshaut. Durch das Abschleifen der Presshaut kann sich die Oberflächenfeuchte der Holzwerkstoffplatte ebenfalls ändern.

Wichtig ist, dass, wie bereits oben angemerkt, diese Randbedingungen, wie Oberfläche mit oder ohne Presshaut, bei der Erstellung des Kalibriermodells berücksichtigt werden.

In einer Ausführungsform des vorliegenden Verfahrens erfolgt die Bestimmung der Oberflächenfeuchte der Holzwerkstoffplatte vor dem Auftrag einer Beschichtung, insbesondere vor dem Auftrag eines Walzgrundes, einer Grundierungsschicht und/oder einer Druckschicht.

So kann die Bestimmung der Oberflächenfeuchte der Holzwerkstoffplatte nach dem Verpressen und Abkühlen der Holzwerkstoffplatte und/oder nach einer Zwischenlagerung und vor dem Einbringen der Holzwerkstoffplatte in eine Produktionslinie zur Beschichtung von Holzwerkstoffplatten erfolgen.

In einer weiteren Ausführungsform des vorliegenden Verfahrens ist von der Oberflächenfeuchte eine Feuchte der Holzwerkstoffplatte bis zu einer Eindringtiefe in die Deckschicht der Holzwerkstoffplatte von bis zu 0,05 mm, bevorzugt von bis 0,1 mm, insbesondere bevorzugt von bis zu 0,15 mm zu verstehen. Insbesondere bezieht sich der zu bestimmende Wert der Oberflächenfeuchte auf eine Feuchte bei einer Eindringtiefe von 0,05 mm - 0,2 mm, bevorzugt 0, 1 mm - 0, 15 mm. Die Dichte der Deckschicht der Holzwerkstoffplatte liegt hierbei zwischen 1000 und 1500 kg/m³, bevorzugt zwischen 1050 und 1300 kg/m³.

In einer weiteren Ausführungsform des vorliegenden Verfahrens ist vorgesehen, dass für die Bestimmung der Korrelation zwischen Oberflächenfeuchte und Benetzbarkeit (bzw. Beschichtbarkeit) ein Benetzungsmittel auf Basis eines wässrigen Harz, bevorzugt eines eingefärbten wässrigen Harzes, verwendet wird. Das wässrige Harz kann ein formaldehydhaltiges Harz, bevorzugt ein Melamin-Formaldehyd-Harz, ein Harnstoff-Formaldehyd-Harz oder ein Gemisch aus beiden sein. Der Feststoffgehalt der Harzschicht liegt zwischen 40 und 70 Gew%, bevorzugt zwischen 45 und 65 Gew%, insbesondere bevorzugt zwischen 50 und 55 Gew%. Es können aber auch wasserhaltige Leime wie PVAc-Leim, wässrige Grundierung auf Basis von Acrylaten oder Isocyanaten zur Anwendung kommen.

Im Rahmen des vorliegenden Verfahrens ist des Weiteren vorgesehen, dass die zu vermessene Holzwerkstoffplatte eine Span-, eine mitteldichte Faser (MDF)-, hochdichte Faser (HDF)-, eine Sperrholzplatte- oder eine Holz-Kunststoff-Komposit-Platte (WPC) ist. Besonders bevorzugt sind Holzfaser- und Holzspanplatten.

Die vorliegende Holzwerkstoffplatte in Form eine Holzspanplatte oder Holzfaserplatte kann eine Rohdichte zwischen 400 und 1200 kg/m³, bevorzugt zwischen 500 und 1000 kg/m³, insbesondere bevorzugt zwischen 600 und 800 kg/m³ aufweisen.

Die Dicke der vorliegenden Holzwerkstoffplatte als Holzspanplatte oder Holzfaserplatte kann zwischen 3 und 20 mm, bevorzugt zwischen 5 und 15 mm betragen, wobei insbesondere eine Dicke von 10 mm bevorzugt ist. Bei der Lackierung von MDF mit Lacken auf Wasserbasis können auch Platten mit Stärken bis zu 50 mm zur Anwendung kommen.

In einer Ausführungsform wird das vorliegende Verfahren in einer Produktionsanlage mit einer Anlagengeschwindigkeit zwischen 400 und 1700 mm/sec, bevorzugt zwischen 500 und 1700 mm/sec durchgeführt.

In einer weitergehenden Ausführungsform des vorliegenden Verfahrens ist vorgesehen, dass der mindestens eine NIR-Messkopf sich quer zur Laufrichtung der Holzwerkstoffplatte über die gesamte Breite des Transportbandes traversiert.

Des Weiteren ist vorteilhafterweise vorgesehen, dass der mindestens eine NIR-Messkopf mehrere Spektren pro Minute, bevorzugt bis zu acht Spektren pro Minute oder mehr aufnimmt, aus denen ein Mittelwert über die Anzahl der aufgenommenen Spektren gebildet wird.

Auch sollte der verwendete NIR-Messkopf unempfindlich gegenüber Temperaturschwankungen, Staub und Emissionen von Holzinhaltstoffen.

Das vorliegende Verfahren ermöglicht die Bereitstellung der Messwerte in kurzer Zeit (online, bevorzugt ohne störende Zeitverzögerung). Die Messdaten können zur Qualitätssicherung, Forschung und Entwicklung, zur Prozesskontrolle, Prozessregelung, Prozesssteuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Wie bereits oben erwähnt, erfolgt die NIR-Messung des vorliegenden Verfahrens zur Bestimmung der Oberflächenfeuchte geeigneter Weise in einer Produktionslinie vor der ersten Vorrichtung (z.B. Walzvorrichtung) zum Auftrag eines Walzgrundes, Grundierungsschicht und/oder Primerschicht; d.h. der NIR-Messkopf ist vor der ersten Auftragsvorrichtung in einer Produktionslinie zur Beschichtung von Holzwerkstoffplatten vorgesehen.

Der NIR-Messkopf ist jeweils mit einem Steuerungssystem mit Auswerteeinheit und Datenbank zur Prozessierung und Speicherung der ermittelten NIR-Daten verbunden. Bei Abweichungen der gemessenen Ist-Werte von den Soll-Werten erfolgt eine automatische Anpassung durch die Anlagensteuerung bzw. -regelung. Grundsätzlich liefern alle in einer Produktionslinie verwendeten NIR-Messköpfe die von ihnen gemessenen Istwerte an die zentrale Steuer- und Auswerte-Einheit liefern, die bei Abweichung der gemessenen Ist-Werte z.B. eines einzigen der NIR-Messköpfe von den entsprechenden Sollvorgaben den Produktionsablauf entsprechend regelt oder vorausschauend steuert.

Es wird somit ein Verfahren bereitgestellt, in welchem durch die Verwendung eines NIR-Messkopfes die Oberflächenfeuchte als Indikator für den Benetzungsgrad bzw. Beschichtbarkeit einer Holzwerkstoffplatte aus einem einzigen NIR-Spektrum bestimmt werden kann, und zwar durch eine berührungslose Messung. Die mit dem Messkopf oder den Messköpfen ermittelten Daten werden in einer vorteilhaften Ausprägung der Erfindung direkt zur Anlagensteuerung oder -regelung verwendet.

So können die mit dem vorliegenden Verfahren ermittelten Parameter der Oberflächenfeuchte und der dazu korrespondierenden Beschichtbarkeit zur Steuerung von mindestens einer Produktionslinie zur Beschichtung von Holzwerkstoffplatten verwendet werden. Insbesondere können die ermittelten Parameter der Oberflächenfeuchte und der dazu korrespondierenden Beschichtbarkeit eine Steuerung der Anlage bezüglich der Produktionsgeschwindigkeit bzw. bei qualitativ nicht einwandfreien Platten eine Ausschleusung von Platten an der Anlage ermöglichen. Im Extremfall sollte auch die Weiterverarbeitung von Platten gestoppt werden
Zudem wird in einer weiteren vorteilhaften Ausprägung der Erfindung durch die Speicherung der Daten eine Verbesserung der Qualitätskontrolle ermöglicht. Durch das sofortige Vorliegen der Messwerte und der hohen Messfrequenz wird eine sehr enge Kontrolle bzw. Steuerung oder Regelung der Anlagen ermöglicht.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Kostenreduzierung und Verbesserung der Anlagenverfügbarkeit.

Das Steuerungssystem der jeweiligen Fertigungsanlage umfasst mindestens eine computergestützte Auswerteeinheit (bzw. Prozessoreinheit) und eine Datenbank. In der Auswerteeinheit erfolgt der Abgleich bzw. Vergleich des für die Holzwerkstoffplatte gemessenen NIR-Spektrums mit den für die jeweils einzelnen Parameter erstellten Kalibriermodellen. Die so bestimmten Parameterdaten werden in der Datenbank gespeichert.

Die mit dem vorliegenden spektroskopischen Verfahren bestimmten Daten können zur Steuerung der jeweiligen Fertigungslinie verwendet werden. Die berührungslos gemessenen Parameterwerte des NIR-Multimesskopfes ("Ist-Werte") können, wie zuvor bereits beschrieben, direkt und in "real time" für die Steuerung bzw. Regelung der betreffenden Anlage verwendet werden, indem beispielsweise die gemessenen und in der Datenbank, z.B. einer relationalen Datenbank, Ist-Werte gespeichert und mit dort vorhandenen Soll-Werten dieser Parameter verglichen werden. Die sich ergebenden Differenzen werden anschließend zur Steuerung bzw. Regelung der Produktionslinie verwendet.

Für den Abgleich und die Steuerung der jeweiligen Fertigungslinie wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der jeweiligen Fertigungslinie gespeichert.

Die Erfindung wird nachfolgend an Ausführungsbeispielen unter Bezugnahme auf die Figuren näher erläutert. Es zeigen:

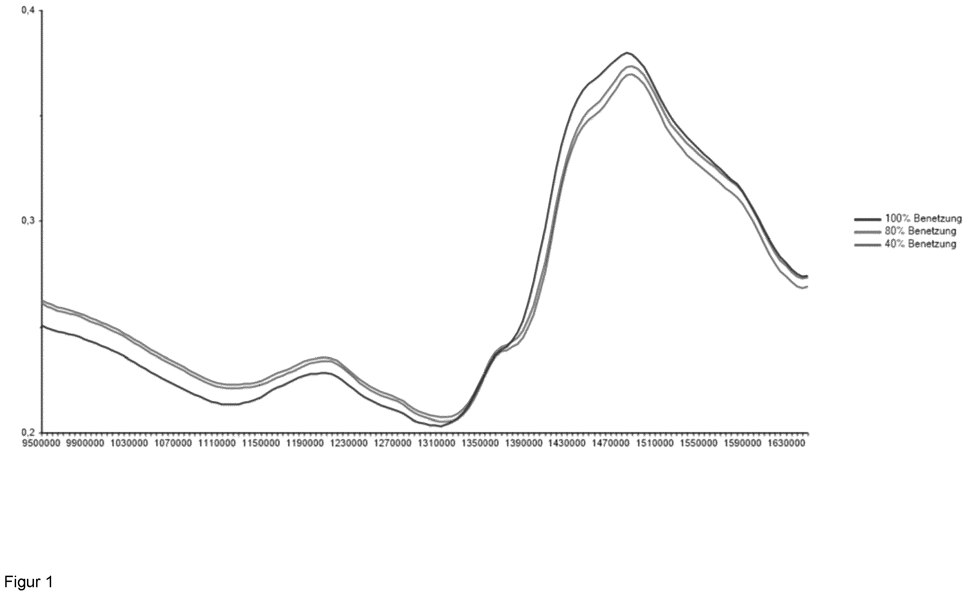

- Figur 1: NIR-Spektren aufgenommen gemäß einer ersten Ausführungsform des vorliegenden Verfahrens; und

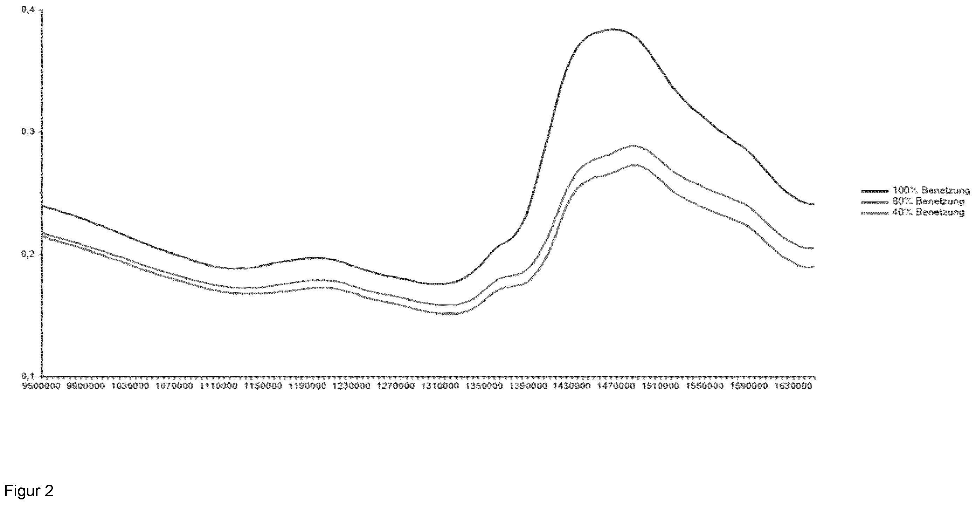

- Figur 2: NIR-Spektren aufgenommen gemäß einer zweiten Ausführungsform des vorliegenden Verfahrens.

### Ausführungsbeispiel 1:

Aus einer 8 mm HDF ( hochdichte Faserplatte ) werden Proben 20 x 20 cm herausgeschnitten. Diese werden in einem Trockenschrank bei 105 °C 24 h getrocknet. Anschließend werden unter Feuchteausschluss abgekühlt, an den Kanten und auf der Rückseite mit Alufolie beklebt und dann gewogen. Dann wird von der nicht mit Alufolie beklebten Seite ein NIR-Spektrum angefertigt.

Ein Satz von drei getrockneten Platten wird dann mit Hilfe eines Rakels mit einem eingefärbtem Melaminharz ( Feststoffgehalt: 55 Gew% ) beschichtet und der Benetzungsgrad bestimmt. Aus den drei Ergebnissen wird ein Mittelwert bestimmt.

Danach werden die weiteren getrockneten Platten ( jeweils drei pro Variante ) mit einem feuchten Lappen unterschiedlich stark befeuchtet, gewogen, jeweils ein NIR-Spektrum angefertigt und dann ebenfalls mit einem Melaminharz mit Hilfe eines Rakels beschichtet. Auch hier wurde jeweils aus drei Werten ein Mittelwert bestimmt.

Die unterschiedlichen Feuchten wurden jeweils durch eine steigende Anzahl von Befeuchtungen mit dem Lappen realisiert. Die Feuchte der Oberfläche wurde dabei auf eine Eindringtiefe von 0,1 bis 0,15 mm und einer Dichte der Deckschicht in diesen 0,1 bis 0,15 mm von 1100 kg/m³ berechnet (siehe Tabelle 1).

**Tabelle 1**

| HDF | Benetzungsgrad (%) | Auftragsmenge Wasser (g) | % Feuchte (bez. auf 0,1-0,15 mm) |
|---|---|---|---|
| 1 | 100 | ca. 16g/m2 | 10,7 - 14,6 |
| 2 | 80 | ca. 8g/m2 | 5,4 - 7,3 |
| 3 | 40 | ca. 4g/m2 | 2,7 - 3,6 |

Von den befeuchteten Proben wurden anschließend NIR-Spektren angefertigt. Dabei zeigte sich, dass beim Wasserpeak zwischen 1400 und 1550 nm in Abhängigkeit von der Feuchte deutliche Unterschiede erkennbar sind.

Anschließend wurde ein Korrelationsmodell erstellt und dieses an Proben mit unbekannter Deckschichtfeuchte aus der Produktion erprobt. Es zeigte sich, dass mit dem Kalibrationsmodell eine präzise Vorhersage bezüglich der Grundierung/Bedruckung gemacht werden konnte.

### Ausführungsbeispiel 2:

An einer 16 mm Spanplatte wurden die gleichen Untersuchungen wie an der HDF durchgeführt. Dabei wurde als Dichte in den 0,1 - 0,15 mm Deckschicht 1000 kg/m³ angenommen.

Im Unterschied zu den HDF-Mustern wurde die Benetzung bei den Spanplattenmustern mit einem eingefärbten Harnstoffharz ( Feststoffgehalt 50 Gew% ) bestimmt. Dabei zeigte sich, dass eine 80 %ige Benetzung bei etwas niedrigeren Feuchtewerten erreicht wurde (siehe Tabelle 2).

**Tabelle 2**

| Spanplatte, 16 mm | Benetzungsgrad (%) | Auftragsmenge Wasser (g) | % Feuchte bez. auf 0,1 - 0,15 mm |
|---|---|---|---|
| 1 | 100 | ca. 16g/m2 | 10,7 - 16,0 |
| 2 | 80 | ca. 5g/m2 | 3,3 - 5,0 |
| 3 | 40 | ca. 3g/m2 | 2 - 3,0 |

Von den getrockneten und dann befeuchteten Proben wurden wiederum NIR-Spektren angefertigt. Diese zeigen ebenso wie die HDF-Proben in Abhängigkeit von der Auftragsmenge Wasser deutliche Unterschiede in den Spektren.

Bei den Spanplattenmustern war ebenfalls bei ca. 80% ein zufriedenstellendes Ergebnis bei der Grundierung/Bedruckung zu erwarten. Nach der Erstellung eines Kalibrationsmodells wurde dies ebenfalls an Proben aus der Produktion mit unbekannter Deckschichtfeuchte erprobt. Auch hier war eine gute Vorhersage der Grundierbarkeit/Bedruckbarkeit möglich. Auch die Güte des Auftrags von Harnstoffleimen an Kaschieranlagen konnte mit dem System erfolgen.

## Patentansprüche

1. Online-Verfahren zur Vorhersage der Benetzbarkeit der Oberfläche mit einem Benetzungsmittel einer Holzwerkstoffplatte, wobei als Parameter für die Benetzbarkeit der Oberfläche der Holzwerkstoffplatte mit dem Benetzungsmittel die Feuchte der Oberfläche mittels NIR-Spektroskopie bestimmt wird und durch Vergleich mit korrespondierenden Benetzungsgraden aus einem mit Referenzproben erstellten Referenzmodell einer Benetzungsbarkeit der Oberfläche der Holzwerkstoffplatte mit dem Benetzungsmittel zugeordnet wird.

2. Verfahren nach Anspruch 1, umfassend die Schritte:
- Bereitstellen von Holzwerkstoffplatten mit jeweils unterschiedlich definierter Oberflächenfeuchte als Referenzproben,
- Aufnahme von mindestens einem NIR-Spektrum von jeder dieser Referenzproben unter Verwendung von mindestens einem NIR-Messkopf in einem Wellenlängenbereich zwischen 1000 nm und 2000 nm, bevorzugt zwischen 1400 nm und 1600, insbesondere bevorzugt zwischen 1400 nm und 1550 nm,
- Zuordnen der unterschiedlichen definierten Oberflächenfeuchte der Referenzproben zu den aufgenommenen NIR-Spektren der jeweiligen Referenzproben,
- Auftragen eines Benetzungsmittels auf die Oberfläche jeder dieser vermessenen Referenzproben mit jeweils unterschiedlich definierter Oberflächenfeuchte und Bestimmen des jeweiligen Benetzungsgrades;
- Erstellen eines Kalibriermodells für den Zusammenhang zwischen den Daten der aufgenommenen NIR-Spektren und den dazugehörigen Oberflächenfeuchten als Maß für die dazu korrespondierenden Benetzungsgrade mittels einer multivariaten Datenanalyse;
- Bereitstellen von mindestens einer Holzwerkstoffplatte mit unbekannter Oberflächenfeuchte;
- Aufnehmen von mindestens einem NIR-Spektrum der mindestens einen Holzwerkstoffplatte mit unbekannter Oberflächenfeuchte unter Verwendung von mindestens einem NIR-Messkopf in einem Wellenlängenbereich zwischen 1000 nm und 2000 nm, bevorzugt zwischen 1400 nm und 1600, insbesondere bevorzugt zwischen 1400 nm und 1550 nm, und
- Bestimmen der Oberflächenfeuchte als Maß für die dazu korrespondierenden Benetzungsgrade durch Vergleich des für die Holzwerkstoffplatte aufgenommenen NIR-Spektrums mit dem erstellten Kalibriermodell.

3. Verfahren nach einem der vorhergehenden Ansprüche, **dadurch gekennzeichnet, dass** die Bestimmung der Oberflächenfeuchte der Holzwerkstoffplatte vor dem Auftrag einer Beschichtung, insbesondere vor dem Auftrag eines Walzgrundes, einer Grundierungsschicht und/oder einer Druckschicht, erfolgt.

4. Verfahren nach einem der vorhergehenden Ansprüche, **dadurch gekennzeichnet, dass** die Oberflächenfeuchte bis zu einer Eindringtiefe in die Holzwerkstoffplatte von bis zu 0,2 mm, bevorzugt von bis 0,15 mm, insbesondere bevorzugt von bis zu 0,15 mm bestimmt wird.

5. Verfahren nach einem der vorhergehenden Ansprüche, **dadurch gekennzeichnet, dass** die zu vermessende Oberfläche der Holzwerkstoffplatte mit einer Presshaut oder Verrottungsschicht versehen ist.

6. Verfahren nach einem der Ansprüche 2-5, **dadurch gekennzeichnet, dass** als Benetzungsmittel ein wässriges Harz, bevorzugt ein eingefärbtes wässriges Harz, wasserhaltige Leime, wie PVAc-Leim, eine wässrige Grundierung auf Basis von Acrylaten oder Isocyanaten, verwendet wird.

7. Verfahren nach einem der vorhergehenden Ansprüche, **dadurch gekennzeichnet, dass** für die Bestimmung der Oberflächenfeuchte Daten aus dem gesamten aufgenommenen Spektralbereich, bevorzugt aus dem aufgenommenen Bereich zwischen 1400 nm und 1600, insbesondere bevorzugt zwischen 1400 nm und 1550 nm, verwendet werden.

8. Verfahren nach einem der vorhergehenden Ansprüche, **dadurch gekennzeichnet, dass** die zu vermessende Holzwerkstoffplatte eine Span-, eine mitteldichte Faser (MDF)-, hochdichte Faser (HDF)- oder eine Sperrholzplatte oder eine Holz-Kunststoff-Komposit-Platte (WPC) ist.

9. Verfahren nach einem der vorhergehenden Ansprüche, **dadurch gekennzeichnet, dass** die Bestimmung der Oberflächenfeuchte der Holzwerkstoffplatte vor dem Einbringen der Holzwerkstoffplatte in eine Produktionslinie zur Beschichtung von Holzwerkstoffplatten erfolgt.

10. Verfahren nach einem der vorhergehenden Ansprüche, **dadurch gekennzeichnet, dass** der mindestens eine NIR-Messkopf sich quer zur Laufrichtung der zu vermessenden Holzwerkstoffplatte bewegt und über die gesamte Breite des Transportbandes traversiert.

11. Verfahren nach einem der vorhergehenden Ansprüche, **dadurch gekennzeichnet, dass** der mindestens eine NIR-Messkopf mehrere Spektren pro Minute, bevorzugt bis zu acht Spektren pro Minute aufnimmt, aus denen ein Mittelwert über die Anzahl der aufgenommenen Spektren gebildet wird.

## Claims

1. An online method for predicting the wettability of the surface of a wood-based panel with a wetting agent, wherein the surface moisture is determined by NIR spectroscopy as a parameter for the wettability of the surface of the wood-based panel with the wetting agent, and is assigned to a wettability of the surface of the wood-based panel with the wetting agent by comparison with corresponding wettability levels from a reference model of the wettability of the surface of the wood-based panel with the wetting agent, created using reference samples.

2. Method according to claim 1, comprising the steps:
- providing wood-based panels, each with a differently defined surface moisture, as reference samples,
- recording at least one NIR spectrum of each of these reference samples using at least one NIR measuring head in a wavelength range between 1000 nm and 2000 nm, preferably between 1400 nm and 1600 nm, and most preferably between 1400 nm and 1550 nm,
- assigning the different defined surface moistures of the reference samples to the recorded NIR spectra of the respective reference samples,
- applying a wetting agent to the surface of each of these measured reference samples, each with a differently defined surface moisture, and determining the respective degree of wetting;
- creating a calibration model for the relationship between the data from the recorded NIR spectra and the corresponding surface moistures as a measure of the corresponding degrees of wetting using multivariate data analysis;
- providing at least one wood-based panel with unknown surface moisture;
- recording at least one NIR spectrum of the at least one wood-based panel with unknown surface moisture using at least one NIR measuring head in a wavelength range between 1000 nm and 2000 nm, preferably between 1400 nm and 1600 nm, and most preferably between 1400 nm and 1550 nm, and
- determining the surface moisture as a measure of the corresponding degrees of wetting by comparing the NIR spectrum recorded for the wood-based panel with the calibration model created.

3. Method according to one of the preceding claims, **characterized in that** the determination of the surface moisture of the wood-based panel is performed prior to the application of a coating, in particular prior to the application of a roller applied primer, a base coat layer, and/or a print layer.

4. Method according to one of the preceding claims, **characterized in that** the surface moisture is determined to a penetration depth into the wood-based panel of up to 0.2 mm, preferably up to 0.15 mm, and most preferably up to 0.15 mm.

5. Method according to one of the preceding claims, **characterized in that** the surface of the wood-based panel to be measured is provided with a press skin or a precured layer.

6. Method according to one of claims 2-5, **characterized in that** an aqueous resin, preferably a colored aqueous resin, water-based adhesives such as PVAc adhesive, or an aqueous primer based on acrylates or isocyanates is used as a wetting agent.

7. Method according to one of the preceding claims, **characterized in that**, for determining the surface moisture, data from the entire recorded spectral range, preferably from the recorded range between 1400 nm and 1600 nm, and more particularly preferably between 1400 nm and 1550 nm, are used.

8. Method according to one of the preceding claims, **characterized in that** the wood-based panel to be measured is a particleboard, a medium-density fiberboard (MDF), a high-density fiberboard (HDF), a plywood panel, or a wood-plastic composite (WPC) panel.

9. Method according to one of the preceding claims, **characterized in that** the determination of the surface moisture of the wood-based panel takes place before the wood-based panel is fed into a production line for coating wood-based panels.

10. Method according to any of the preceding claims, **characterized in that** the at least one NIR measuring head moves transversely to the direction of travel of the wood-based panel to be measured and traverses the entire width of the conveyor belt.

11. Method according to one of the preceding claims, **characterized in that** the at least one NIR measuring head records several spectra per minute, preferably up to eight spectra per minute, from which an average value is calculated based on the number of recorded spectra.

## Revendications

1. Procédé en ligne de prédiction de la mouillabilité de la surface d'un panneau en matériau dérivé du bois avec un agent mouillant, dans lequel l'humidité de la surface est déterminée par spectroscopie NIR en tant que paramètre de la mouillabilité de la surface du panneau en matériau dérivé du bois et est associée à une mouillabilité de la surface du panneau en matériau dérivé du bois avec l'agent mouillant par comparaison à des degrés de mouillage correspondants issus d'un modèle de référence créé avec des échantillons de référence.

2. Procédé selon la revendication 1, comprenant les étapes :
- de mise à disposition de panneaux en matériau dérivé du bois avec une humidité de surface définie respectivement différemment en tant qu'échantillons de référence,
- d'enregistrement d'au moins un spectre NIR de chacun des échantillons de référence en utilisant au moins une tête de mesure NIR dans une plage de longueurs d'onde entre 1000 nm et 2000 nm, et de manière préférée entre 1400 nm et 1600 nm, en particulier de manière préférée entre 1400 nm et 1550 nm,
- d'association de l'humidité de surface différente définie des échantillons de référence aux spectres NIR enregistrés des échantillons de référence respectifs,
- d'application d'un agent mouillant sur la surface de chacun desdits échantillons de référence mesurés avec une humidité de surface définie respectivement différemment et de détermination du degré de mouillage respectif ;
- de création d'un modèle d'étalonnage pour la corrélation entre les données des spectres NIR enregistrés et les humidités de surface associées comme mesure des degrés de mouillage correspondants au moyen d'une analyse de données à variables multiples ;
- de mise à disposition d'au moins un panneau en matériau dérivé du bois avec une humidité de surface inconnue ;
- d'enregistrement d'au moins un spectre NIR de l'au moins un panneau en matériau dérivé du bois avec une humidité de surface inconnue en utilisant au moins une tête de mesure NIR dans une plage de longueurs d'onde entre 1000 nm et 2000 nm, de manière préférée entre 1400 nm et 1600 nm, en particulier de manière préférée entre 1400 nm et 1550 nm, et
- de détermination de l'humidité de surface en tant que mesure des degrés de mouillage correspondants en comparant le spectre NIR enregistré pour le panneau en matériau du bois au modèle d'étalonnage créé.

3. Procédé selon l'une quelconque des revendications précédentes, **caractérisé en ce que** la détermination de l'humidité de surface du panneau en matériau dérivé du bois est effectuée avant l'application d'un revêtement, en particulier avant l'application d'une primaire au rouleau, d'une couche d'apprêt et/ou d'une couche d'impression.

4. Procédé selon l'une quelconque des revendications précédentes, **caractérisé en ce que** l'humidité de surface est déterminée jusqu'à une profondeur de pénétration dans le panneau en matériau dérivé du bois allant jusqu'à 0,2 mm, de manière préférée jusqu'à 0,15 mm, en particulier de manière préférée jusqu'à 0,15 mm.

5. Procédé selon l'une quelconque des revendications précédentes, **caractérisé en ce que** la surface à mesurer du panneau en matériau dérivé du bois est pourvue d'une pellicule de pressage ou d'une couche de décomposition.

6. Procédé selon l'une quelconque des revendications 2 à 5, **caractérisé en ce qu'**une résine aqueuse, de manière préférée une résine aqueuse colorée, des colles contenant de l'eau, comme une colle PVAc, un apprêt aqueux à base d'acrylates ou d'isocyanates, sont utilisés en tant qu'agent mouillant.

7. Procédé selon l'une quelconque des revendications précédentes, **caractérisé en ce que** des données issues l'ensemble de la plage spectrale enregistrée, de manière préférée issues de la plage enregistrée comprise entre 1400 nm et 1600, en particulier de manière préférée entre 1400 nm et 1550 nm sont utilisées pour déterminer l'humidité de surface.

8. Procédé selon l'une quelconque des revendications précédentes, **caractérisé en ce que** le panneau en matériau dérivé du bois à mesurer est un panneau de particules, un panneau de fibres à densité moyenne (MDF), un panneau de fibres à densité élevée (HDF) ou un panneau en contreplaqué ou un panneau composite bois-plastique (WPC).

9. Procédé selon l'une quelconque des revendications précédentes, **caractérisé en ce que** la détermination de l'humidité de surface du panneau en matériau dérivé du bois est effectuée avant l'introduction du panneau en matériau dérivé du bois dans une ligne de production pour le revêtement de panneaux en matériau dérivé du bois.

10. Procédé selon l'une quelconque des revendications précédentes, **caractérisé en ce que** l'au moins une tête de mesure NIR se déplace transversalement par rapport au sens de défilement du panneau en matériau du bois à mesurer et traverse toute la largeur de la bande transporteuse.

11. Procédé selon l'une quelconque des revendications précédentes, **caractérisé en ce que** l'au moins une tête de mesure NIR enregistre plusieurs spectres par minute, de manière préférée jusqu'à huit spectres par minute, à partir desquels une valeur moyenne sur le nombre des spectres enregistrés est obtenue.
